# EUROPÄISCHE PATENTSCHRIFT

(11) **EP 0 717 963 B1**
(45) Veröffentlichungstag und Bekanntmachung des Hinweises auf die Patenterteilung: **08.03.2000**
(21) Anmeldenummer: 95120526.9
(22) Anmeldetag: 27.12.1995
(51) Int. Cl.: A61B 17/30

(54) **Vorrichtung zum Entfernen von Zecken**
Device for removing ticks
Dispositif pour oter les tiques

(30) Priorität: 24.12.1994 DE 4446762
(43) Veröffentlichungstag der Anmeldung: 26.06.1996
(73) Patentinhaber: FORTUNA-VERTRIEB Dr. G. Schick GmbH, 74889 Sinsheim (DE)
(72) Erfinder: Schick, Gerhard, Dr., D-74889 Sinsheim (DE)
(74) Vertreter: Naumann, Ulrich, Dr.-Ing.

(56) Entgegenhaltungen:
- DE-U- 9 109 361
- FR-A- 2 483 770
- US-A- 5 116 347

## Beschreibung

Die vorliegende Erfindung betrifft eine Vorrichtung zum Entfernen von Zecken mit zwei jeweils eine Greifbacke aufweisenden, zumindest teilweise elastischen Greifarmen, wobei die Greifarme einander zugeordnet und die Greifbacken aufgrund der Elastizität der Greifarme aufeinander zu und voneinander weg bewegbar sind und wobei sich die Greifarme zwischen einem den Greifbacken abgewandten Verbindungsbereich und den Greifbacken derart kreuzen, daß die Greifbacken mit ihren Eingriffsflächen unter Vorspannung aneinanderliegen und das so gebildete Maul durch Überwindung der Vorspannung öffenbar ist,

Vorrichtungen der in Rede stehenden Art sind seit langem, beispielsweise in Form von Pinzetten, aus der Praxis bekannt und existieren in den unterschiedlichsten Ausführungsformen und -größen. Derartige Vorrichtungen finden ihre Verwendung sowohl im privaten als auch im beruflichen Bereich immer dann, wenn sehr kleine Objekte zu greifen oder handzuhaben sind und ein Anfassen bzw. Greifen mittels der Finger aufgrund der Größe oder der Empfindlichkeit des zu greifenden Objekts nicht möglich ist.

Oftmals werden Pinzetten bei der Entfernung von Zecken von der menschlichen oder tierischen Haut während des Blutsaugens der Zecken verwendet. Eine solche Entfernung von Zecken von der Haut ist erforderlich, da durch den Stich der Zecken gefährliche Krankheiten auf Mensch und Haustiere übertragen werden können. Sowohl beim Entfernen mittels der Finger als auch mittels einer Pinzette läßt sich meist nur der beim Saugakt stark anschwellende Hinterleib der Zecke abreißen, während der Kopf der Zecke nach wie vor auf der Haut anhaftet bzw. in dieser steckt. Die Übertragung von Krankheiten ist in diesem Zustand in unverminderter Weise möglich.

Eine vollständige Entfernung einer Zecke während des Blutsaugens läßt sich meist nur durch ein gefühlvolles Greifen bzw. Festhalten und anschließendes Herausdrehen des Zeckenkopfes erreichen.

Eine herkömmliche Pinzette ist bei dem Herausdrehen einer Zecke problematisch, da eine Drehbewegung bei gleichzeitigem Festhalten der Zecke nur entlang eines kleinen Drehwinkelbereichs möglich ist, ohne an der Pinzette umgreifen zu müssen, wozu beide Hände erforderlich sind. Während des Umgreifens wird die Zecke oftmals aufgrund einer unbeabsichtigten Druckentlastung der elastischen Greifarme losgelassen und muß dann zusätzlich zu einem weiteren Drehen wieder neu gegriffen werden. Nicht selten wird die Zecke bei dieser Manipulation zerquetscht, woraus eine verschlechterte Greifbarkeit und damit eine weitere Erschwerung oder gar ein Unmöglichwerden der Entfernung der Zecke resultiert. Folglich ist das Entfernen einer Zecke mittels einer herkömmlichen Pinzette äußerst umständlich und häufig völlig unmöglich.

Die FR-A-2 483 770 offenbart eine Vorrichtung zum Entfernen von Zecken mit allen Merkmalen des Oberbegriffs des Patentanspruchs 1. Dabei ist die Vorrichtung in Form einer Pinzette ausgebildet. Eine derartige Pinzette ist, wie oben erwähnt, beim Herausdrehen einer Zecke problematisch, da hier entsprechend umgegriffen werden muß.

Des weiteren ist aus der US-A-5 116 347 eine zangenartige Vorrichtung zum Entfernen von Zecken bekannt. Die Greifbacken der bekannten Vorrichtung liegen nicht unter Vorspannung aneinander. Die Greifbacken sind derart ausgebildet, daß sich ein hinreichend großer Hohlraum für den Hinterleib der Zecke im gegriffenen Zustand ergibt.

Der vorliegenden Erfindung liegt daher die Aufgabe zugrunde, eine Vorrichtung zum Entfernen von Zecken der eingangs genannten Art derart auszugestalten und weiterzubilden, daß die Handhabung, d.h. Ansetzen sowie Herausdrehen der Zecke, vereinfacht ist.

Erfindungsgemäß wird die voranstehende Aufgabe durch die Merkmale des Patentanspruches 1 gelöst. Danach ist die in Rede stehende Vorrichtung derart ausgebildet, daß die Greifarme zumindest in dem Verbindungsbereich von einer ein- oder mehrteiligen zylinderförmigen Hülse umgeben sind und daß die Hülse den Betätigungsbereich umgibt und in ihrer Wandung Öffnungen zum Aufnehmen und/oder Betätigen der Betätigungselemente aufweist.

Zur Entfernung der Zecken wird in einfacher Weise das Maul der Vorrichtung geöffnet und werden die Eingriffsflächen der Greifbacken unter Wirkung der Vorspannung mit dem Zeckenkopf in Eingriff gebracht. Dieser Eingriff zwischen der Vorrichtung und der Zecke bleibt aufgrund der Vorspannung auch während weiterer Manipulationen bzw. während eines sich anschließenden Herausdrehens der Zecke erhalten, wobei die Zecke stets mit derselben Kraft beaufschlagt ist.

Zum Ermöglichen einer besonders einfachen Handhabung der Vorrichtung sind die Greifarme zumindest in dem Verbindungsbereich von einer Hülse umgeben. Eine solche Hülse vereinfacht nicht nur ein Angreifen der Vorrichtung, sondern auch das Rollen der Vorrichtung zwischen zwei Fingern während des Herausdrehens der Zecke. Eine besonders homogene Ausgestaltung der Vorrichtung ist dadurch erreicht, daß die Hülse auch den Betätigungsbereich umgibt und in ihrer Wandung Öffnungen zum Aufnehmen und/oder Betätigen der Betätigungselemente aufweist. In praktischer Weise ragen dabei die Betätigungselemente durch die Hülsenwandung hindurch, wodurch neben einem einfachen Angreifen der Vorrichtung auch ein einfaches Betätigen der Betätigungselemente ermöglicht ist. Jedoch auch wenn die Betätigungselemente nicht durch die Hülsenwandung hindurchragen, können die Öffnungen derart ausgebildet sein, daß ein Betätigen der Betätigungselemente von außen durch die Öffnungen hindurch ermöglicht ist.

Aufgrund der sehr einfachen Handhabung der erfindungsgemäßen Vorrichtung ist insbesondere die Entfernung von Zecken an Tieren unproblematisch. Dabei kann das sich gegebenenfalls bewegende Tier mit einer Hand festgehalten werden, während die Zecke mit der anderen Hand durch Rollen der Vorrichtung zwischen zwei Fingern herausgedreht werden kann.

Im Hinblick auf eine möglichst einfache Betätigung der Vorrichtung könnte zwischen dem Verbindungsbereich und dem Kreuzungsbereich der Greifarme ein Betätigungsbereich mit jeweils einem von den Greifarmen abragendem Betätigungselement vorgesehen sein. Eine besonders wirksame Betätigung der Vorrichtung könnte dadurch realisiert sein, daß die Betätigungselemente in entgegengesetzter Richtung im wesentlichen innerhalb der Bewegungsebene der Greifbacken von den Greifarmen abragen. Mittels einer derartigen Ausbildung wäre das durch die Greifbacken gebildete Maul auf einfache Weise durch ein aufeinander zu gerichtetes Drücken der Betätigungselemente, beispielsweise mit zwei Fingern, ermöglicht. Unwirksame Kraftkomponenten außerhalb der Bewegungsebene der Greifbacken wären damit während des Öffnens des Mauls vermieden.

In besonders einfacher Weise könnten die Betätigungselemente als integrale Bestandteile der Greifarme ausgebildet sein. Die Betätigungselemente könnten dabei direkt zwischen dem Verbindungsbereich und dem Kreuzungsbereich der Greifarme angeordnet sein.

Im Hinblick auf eine einfache Herstellung der Betätigungselemente und damit des Greifarms insgesamt könnten die Betätigungselemente etwa halbringförmig ausgebildet sein. Bei einer derartigen Ausgestaltung wäre eine Herstellung durch ein einfaches Biegen des Greifarms möglich.

Zur Gewährleistung einer leichtgängigen und verschleißarmen Funktion der Vorrichtung könnten sich die Greifarme beim Bewegen der Greifbacken gegebenenfalls berührungslos im Kreuzungsbereich aneinander vorbeibewegen.

In weiter vorteilhafter Weise könnten sich die Greifarme im Bereich zwischen dem Verbindungsbereich und dem Betätigungsbereich gegeneinander abstützen. Insoweit wäre gewährleistet, daß auch bei der Verwendung von Kunststoffen eine hinreichende Steifigkeit bzw. material- und dimensionsspezifische Federkraft beim Biegen der Greifarme auftritt. Jedenfalls ist durch die gegenseitige Abstützung im Bereich zwischen dem Verbindungsbereich und dem Betätigungsbereich gewährleistet, daß die zwischen der Abstützung und den Greifbacken verbleibende Strecke der Greifarme zur Gewährleistung einer ausreichenden Vorspannung beim Öffnen der Greifbacken hinreichend kurz ist.

Im konkreten könnten den beiden Greifarmen jeweils ein Stützelement zum gegenseitigen Abstützen zugeordnet sein. Das Stützelement könnte wiederum in Form eines Stegs ausgebildet sein, wobei der Steg des einen Greifarms am Steg des anderen Greifarms zur Anlage kommt. Da bei der Betätigung der Vorrichtung die Greifarme in ihrem Winkel zueinander veränderbar werden, ist es von ganz besonderem Vorteil, wenn die Stege zumindest geringfügig schwenkbar ineinander eingreifen. Unter Zugrundelegung der voranstehend genannten konstruktiven Merkmale bietet sich eine ein- oder zweiteilige Ausgestaltung an, wobei das Teil bzw. die Teile aus Kunststoff hergestellt sein können. Eine spritzgußtechnische Herstellung sowohl der Greifarme als auch der die Greifarme aufnehmenden Hülse mit sämtlichen weiteren Bestandteilen ist möglich.

Zum Erreichen eines präzisen Eingriffs der Greifbacken bzw. der Eingriffsflächen an dem Zeckenkopf, ohne ein Zerquetschen des gegebenenfalls bereits erheblich angeschwollenen Hinterteils der Zecke, könnten die Greifbacken im wesentlichen von dem Kreuzungsbereich aus in Richtung Eingriffsflächen divergieren und etwa im Bereich der Eingriffsflächen konvergieren. Des weiteren wäre in diesem Zusammenhang günstig, wenn die Greifbacken zwischen dem divergierenden und dem konvergieren-den Bereich einen im unbetätigten Zustand der Vorrichtung etwa parallel zu der jeweils anderen Greifbacke angeordneten Abschnitt aufweisen würden. Auf diese Weise wären die Greifbacken im vorderen Bereich der Vorrichtung teilweise derart mit Abstand zueinander angeordnet, daß genügend Raum für das Hinterteil der Zecke vorhanden und ein Zerquetschen der Zecke vermieden wäre. Hierfür wäre es weiterhin besonders vorteilhaft, wenn die Eingriffsflächen unter Bildung eines Winkels aneinanderliegen würden.

Zur Gewährleistung eines sicheren Eingriffs zwischen Vorrichtung und Zecke könnten die Eingriffsflächen rechteckig oder mehreckig ausgebildet sein. Dabei ist besonders wesentlich, daß die Eingriffsfläche nicht mit einer Ecke, sondern mit einer flachen Seite in Richtung zu greifendes Objekt orientiert ist. Mit einer solchen flachen Seite könnte die Vorrichtung direkt vor dem Greifen der Zecke zur Stabilisierung der Vorrichtung an der Haut angelegt werden. Des weiteren stehen die Eingriffsflächen bei einer derartigen Anlage an der Haut mit ihrer gesamten Breite zum Eingriff zur Verfügung. Erfolglose Greifversuche lassen sich dadurch vermeiden.

Im Hinblick auf eine stabile Verbindung bzw. Zuordnung der Greifarme könnten die Greifarme im Verbindungsbereich parallel aneinander anlegbare Flächen aufweisen. Hierfür könnten sich die Flächen von dem den Greifbacken abgewandten Ende bis zu dem Betätigungsbereich erstrecken.

Hinsichtlich einer besonders einfachen und kostengünstigen Herstellung der Vorrichtung insgesamt könnten die zwei Greifarme identisch ausgebildet sein.

Im Hinblick auf eine noch homogenere Ausgestaltung der Vorrichtung könnte die Hülse ebenfalls die Greifbacken teilweise umgeben.

Eine besondere Vielseitigkeit der Ausgestaltung könnte durch eine mehrteilige Ausbildung der Hülse erreicht werden.

Zum Schutz der Greifbacken bei Nichtbenutzung der Vorrichtung könnte die Hülse eine die Greifbacken umgebende Schutzkappe aufweisen. Zur Verhinderung eines Verschmutzens des Inneren der Hülse bzw. der Greifarme könnte die Hülse an dem den Greifbacken abgewandten Ende eine Abschlußkappe aufweisen.

Im Hinblick auf einen besonders einfachen Zusammenbau bzw. auf ein besonders einfaches Zerlegen der Vorrichtung, beispielsweise zu Reinigungszwecken, könnten die einzelnen Bestandteile der Hülse miteinander verschraubbar, verrastbar oder verklemmbar sein.

Zur Realisierung einer besonders leichten Ausbildung der Vorrichtung könnte die Hülse aus Kunststoff gebildet sein. In einer besonders edlen Version könnte die Hülse auch aus Metall bzw. Edelmetall bestehen. In gleicher Weise könnten auch die Greifarme aus Kunststoff oder Metall gebildet sein.

Eine besonders günstige Anordnung der Greifarme zueinander sowie Anordnung innerhalb der Hülse könnte durch eine Festlegung der Greifarme in ihrem Verbindungsbereich an der Hülse realisiert sein. Hierfür könnten die Greifarme innerhalb zweier in der Abschlußkappe ausgebildeter Öffnungen festgelegt sein.

In besonders einfacher Weise könnten die Greifarme dadurch festgelegt sein, daß einerseits die Greifarme innerhalb der in der Abschlußkappe ausgebildeten Öffnungen angeordnet sind und andererseits die Betätigungselemente aus den in der Hülsenwandung ausgebildeten Öffnungen herausragen, wobei sich die Greifarme sowohl mittels parallel aneinander anlegbarer Flächen im Verbindungsbereich als auch mittels der Eingriffsflächen berühren. Auf diese Weise wären die Greifarme reversibel in der Hülse verklemmt und damit sicher festgelegt. Eine Betätigung der Vorrichtung kann dabei problemlos durch Betätigen der Betätigungselemente von außerhalb der Hülse erfolgen.

Eine besonders stabile Festlegung der Greifarme in der Hülse könnte jedoch auch mittels eines spritzgußtechnischen Ausgießens der Hülse im Verbindungsbereich realisiert werden. Öffnungen innerhalb einer Abschlußkappe wären dann nicht mehr erforderlich.

Es gibt nun verschiedene Möglichkeiten, die Lehre der vorliegenden Erfindung in vorteilhafter Weise auszugestalten und weiterzubilden. Dazu ist einerseits auf die dem Patentanspruch 1 nachgeordneten Ansprüche, andererseits auf die nachfolgende Erläuterung dreier Ausführungsbeispiele der Erfindung anhand der Zeichnung zu verweisen. In Verbindung mit der Erläuterung der bevorzugten Ausführungsbeispiele der Erfindung anhand der Zeichnung werden auch im allgemeinen bevorzugte Ausgestaltungen und Weiterbildungen der Lehre erläutert. In der Zeichnung zeigen
- Fig. 1: in einer Seitenansicht ein Ausführungsbeispiel einer erfindungsgemäßen Vorrichtung,
- Fig. 2: in einer Seitenansicht sowie in einer Draufsicht ein zweites Ausführungsbeispiel eines erfindungsgemäßen Greifarmes,
- Fig. 3: in einer Seitenansicht das Ausführungsbeispiel aus Fig. 1, jedoch ohne Hülse,
- Fig. 4: in einem Längsschnitt sowie in einer Seitenansicht von hinten ein Ausführungsbeispiel einer erfindungsgemäßen Hülse, und
- Fig. 5: in einer Seitenansicht ein weiteres Ausführungsbeispiel einer erfindungsgemäßen Vorrichtung, ebenfalls ohne Hülse.

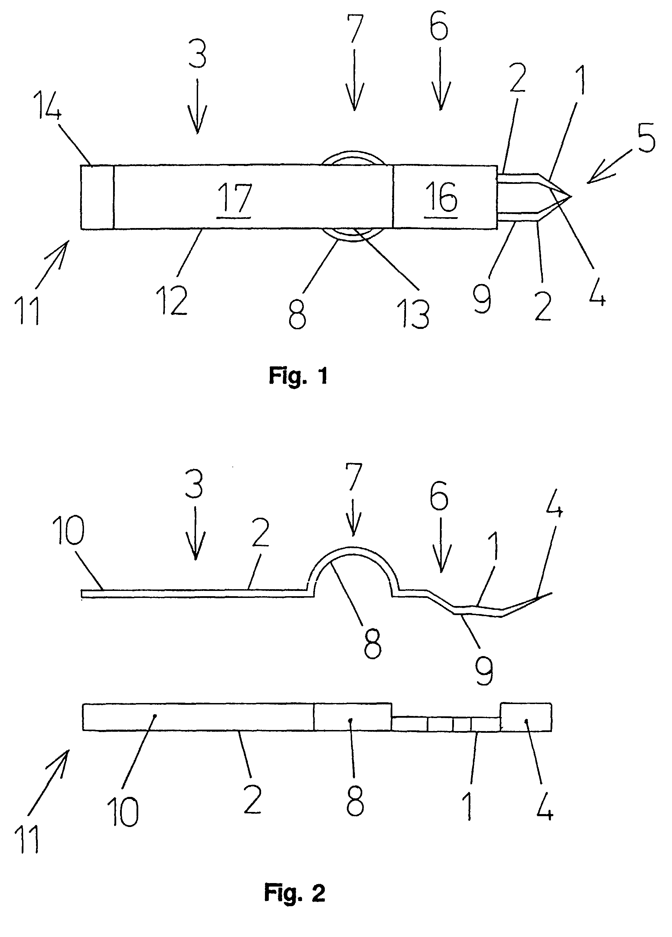

Fig. 1 zeigt in einer Seitenansicht ein erstes Ausführungsbeispiel einer erfindungsgemäßen Vorrichtung zum Entfernen von Zecken. Die Vorrichtung weist zumindest teilweise elastische Greifarme 2 mit jeweils einer Greifbacke 1 auf. Die Greifarme 2 sind dabei einander in einer vorgegebenen Wirkstellung zugeordnet. Die Greifbacken 1 sind aufgrund der Elastizität der Greifarme 2 aufeinander zu und voneinander weg bewegbar. Die Elastizität kann entweder entlang des gesamten Greifarms 2 vorliegen oder auch nur auf Teilbereiche des Greifarms 2 beschränkt sein. Die Greifarme 2 kreuzen sich zwischen einem den Greifbacken 1 abgewandten Verbindungsbereich 3 und den Greifbacken 1 derart, daß die Greifbacken 1 mit ihren Eingriffsflächen 4 unter Vorspannung aneinanderliegen und das so gebildete Maul 5 durch Überwindung der Vorspannung öffenbar ist. Der im Kreuzungsbereich 6 befindliche Kreuzungspunkt ist bei dem in Fig. 1 gezeigten Ausführungsbeispiel nicht sichtbar, da die Greifarme 2 sowohl in dem Verbindungsbereich 3 als auch in dem Kreuzungsbereich 6 von einer Hülse 12 umgeben sind. Ebenfalls von der Hülse 12 umgeben ist ein zwischen dem Verbindungsbereich 3 und dem Kreuzungsbereich 6 der Greifarme 2 ausgebildeter Betätigungsbereich 7. In dem Betätigungsbereich 7 weisen die Greifarme 2 jeweils ein von den Greifarmen 2 abragendes Betätigungselement 8 auf. Das Betätigungselement 8 ragt durch eine in der Wandung der Hülse 12 ausgebildete Öffnung 13 hindurch, so daß es von außerhalb der Hülse 12 betätigbar ist. Durch Betätigen des Betätigungselements 8 wird die Vorspannung der Greifarme 2 überwunden und das Maul 5 geöffnet.

Wesentlich für einen effektiven Öffnungsvorgang des Mauls 5 ist ein Abragen der Betätigungselemente 8 in entgegengesetzter Richtung und zwar im wesentlichen innerhalb der Bewegungsebene der Greifbacken 1.

Bei dem in Fig. 1 gezeigten Ausführungsbeispiel sind die Betätigungselemente 8 als integrale Bestandteile der Greifarme 2 ausgebildet und weisen eine etwa halbringförmige Form auf.

Die Greifbacken 1 divergieren im wesentlichen von dem in Fig. 1 nicht sichtbaren Kreuzungsbereich 6 aus in Richtung Eingriffsflächen 7 und konvergieren etwa im Bereich der Eingriffsflächen 4. Zwischem dem divergierenden und dem konvergierenden Bereich weisen die Greifbacken 1 einen im unbetätigten Zustand der Vorrichtung etwa parallel zu der jeweils anderen Greifbacke 1 ausgebildeten Abschnitt 9 auf. Die Eingriffsflächen 4 liegen unter Bildung eines Winkels aneinander. Dadurch ist gewährleistet, daß beim Greifen einer Zecke das von der Haut abragende Hinterteil der Zecke nicht zerquetscht wird. Die Eingriffsflächen 4 können rechteckig oder mehreckig ausgebildet sein.

Die bei dem gezeigten Ausführungsbeispiel identisch ausgebildeten Greifarme 2 sind von einer aus einem vorderen Teil 16, einem hinteren Teil 17 sowie einer Abschlußkappe 14 aufgebauten Hülse 12 umgeben. Die Abschlußkappe 14 deckt die Hülse 12 am hinteren Ende 11 ab. Das vordere Teil 16 umgibt die Greifbacken 1 bis zu einem Ausmaß, bei dem ein unbeeinträchtigter Einsatz der Greifbacken 1 möglich ist. Die Hülse 12 kann gegebenenfalls eine die Greifbacken 1 umgebende Schutzkappe aufweisen.

Die einzelnen Bestandteile 14, 16 und 17 der Hülse 12 sind im Hinblick auf einen einfachen Zusammenbau und auf ein einfaches Zerlegen, beispielsweise zu Reinigungszwecken, miteinander verschraubbar, verrastbar oder verklemmbar. Die Hülse 12 ist zylinderförmig und besteht aus Metall oder Kunststoff.

Zur sicheren Anordnung der Greifarme 2 in der Hülse 12 ragen die Greifarme 2 einerseits in hier nicht gezeigte in der Abschlußkappe 14 ausgebildete Öffnungen 15 und andererseits mit den Betätigungselementen 8 aus den Öffnungen 13 der Hülse 12 heraus. Durch die Festlegung der Greifarme 2 in den Öffnungen 13 und 15 und eine gleichzeitige Berührung der Greifarme 2 miteinander an parallel aneinander anlegbaren Flächen 10 im Verbindungsbereich 3 und an den Eingriffsflächen 4 sind die Greifarme 2 reversibel in der Hülse 12 festgeklemmt. Auf diese Weise ist eine sichere Aufnahme der Greifarme 2 in der Hülse 12 erreicht.

Die Greifarme 2 könnten jedoch auch vorzugsweise spritzgußtechnisch innerhalb der Hülse 12 vergossen und dadurch festgelegt sein. Eine Reversibilität wäre dann jedoch nicht mehr gewährleistet.

Fig. 2 zeigt sowohl in einer Seitenansicht als auch in einer Draufsicht ein zweites Ausführungsbeispiel eines erfindungsgemäßen Greifarms 2. Der in Fig. 2 gezeigte Greifarm 2 weist im Verbindungsbereich 3 beginnend am hinteren Ende 11 eine parallel an einen anderen Greifarm 2 anlegbare Fläche 10 auf. Zur Funktion der Fläche 10 sei auf die zu der Fig. 1 gehörenden Beschreibung verwiesen.

Direkt an den Verbindungsbereich 3 schließt sich ein Betätigungsbereich 7 mit einem von dem Greifarm 2 abragendem Betätigungselement 8 an. Das Betätigungselement 8 ist als integraler Bestandteil des Greifarms 2 etwa halbringförmig ausgebildet.

Anhand der in der oberen Hälfte der Fig. 2 dargestellten Seitenansicht des Greifarms 2 ist die Form der Greifbacke 1 erkennbar. Im wesentlichen von dem sich an den Betätigungsbereich 7 anschließenden Kreuzungsbereich 6 aus divergiert die Greifbacke 1 bis zu einem parallel zu der Fläche 10 ausgebildeten Abschnitt 9. Der Abschnitt 9 ist im unbetätigten Zustand der Vorrichtung etwa parallel zu der jeweils anderen Greifbacke 1 angeordnet.

Zur Gewährleistung eines möglichst großen Spielraums zwischen den einander zugeordneten Greifarmen 2 divergiert die Greifbacke 1 im Anschluß an den Abschnitt 9 weiter. Etwa im Bereich der Eingriffsflächen 4 konvergiert die Greifbacke 1.

In der in der unteren Hälfte der Fig. 2 dargestellten Draufsicht des Greifarms 2 ist die rechteckige Form der Eingriffsfläche 4 erkennbar. Der Greifarm 2 weist zwischen dem Betätigungselement 8 und der Eingriffsfläche 4 eine Ausnehmung auf, innerhalb derer der andere Greifarm 2 während der Bewegung der Greifbacken 1 führbar ist.

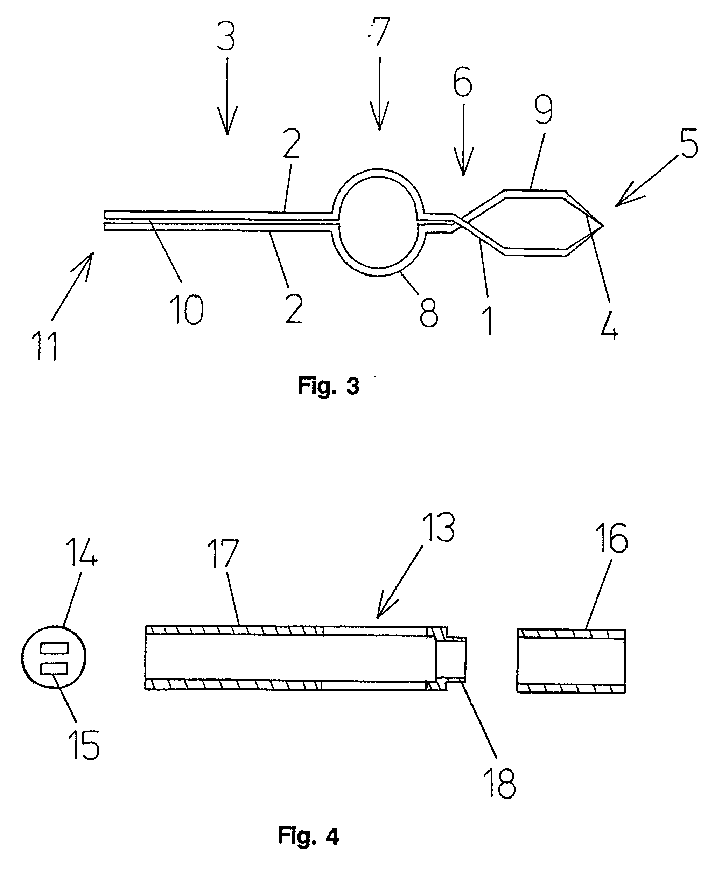

Fig. 3 zeigt in einer Seitenansicht, geringfügig vergrößert, das Ausführungsbeispiel aus Fig. 1, jedoch ohne Hülse 12. Die identisch ausgebildeten Greifarme 2 sind einander derart zugeordnet, daß die sich von dem hinteren Ende 11 bis zum Betätigungsbereich 7 im Verbindungsbereich 3 erstreckenden parallel aneinander anlegbaren Flächen 10 einander gegenüberliegen und gegebenenfalls in Kontakt gelangen können. Die Betätigungselemente 8 sind derart ausgebildet, daß sie durch die Hülse 12 hindurchragen können. Im Kreuzungsbereich 6 kreuzen sich die Greifarme 2. Die Greifbacken 1 bilden mit ihren divergierenden, parallelen und konvergierenden Bereichen genügend Raum zur Aufnahme eines angeschwollenen Hinterteils einer Zecke. Die Eingriffsflächen des so gebildeten Mauls 5 liegen unter Vorspannung aneinander.

Fig. 4 zeigt in einem Längsschnitt das vordere Teil 16 und das hintere Teil 17 einer Hülse 12 sowie in einer Seitenansicht von hinten eine Abschlußkappe 14 mit darin ausgebildeten Öffnungen 15 zur Aufnahme von Greifarmen 2. Das hintere Hülsenteil 17 weist ein Rastelement 18 zum Eingriff mit dem vorderen Teil 16 sowie zwei Öffnungen 13 für die Betätigungselemente 8 auf. Sämtliche Hülsenteile 14, 16 und 17 sind miteinander verschraubbar, verrastbar oder verklemmbar.

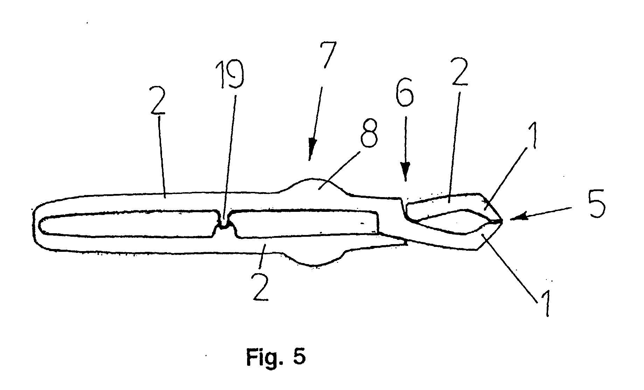

Fig. 5 zeigt ein weiteres Ausführungsbeispiel einer erfindungsgemäßen Vorrichtung, wobei sich dort die Greifarme 2 im Bereich zwischen dem Verbindungsbereich 3 und dem Betätigungsbereich 7 gegeneinander abstützen. Insoweit wird die Steifigkeit in dem beim Öffnen der Vorrichtung verbiegbaren Bereich zwischen der Abstützung und den Greifbacken 1 erhöht. Fig. 5 zeigt des weiteren, daß den beiden Greifarmen 2 jeweils ein Stützelement 19 zum gegenseitigen Abstützen zugeordnet ist, wobei dieses Stützelement 19 in Form eines Stegs ausgebildet ist, der im konkreten etwa orthogonal von den Greifarmen 2 nach innen abragt. Des weiteren ist bei dem hier gewählten Ausführungsbeispiel wesentlich, daß der Steg des einen Greifarms 2 am Steg des anderen Greifarms 2 zur Anlage kommt, wobei die Stege zumindest geringfügig schwenkbar ineinander eingreifen. Zur Vervollständigung der Vorrichtung wird - wie bei den zuvor erörterten Ausführungsbeispielen - eine Hülse darübergeschoben, wobei lediglich der Betätigungsbereich 7 zugänglich sein muß.

Hinsichtlich weiterer vorteilhafter Ausbildungen der Vorrichtung zum Entfernen von Zecken wird zur Vermeidung von Wiederholungen auf den allgemeinen Teil der Beschreibung sowie auf die nachgeordneten Patentansprüche verwiesen.

Abschließend sei hervorgehoben, daß die voranstehend lediglich beispielhaft genannten Ausführungsbeispiele der erfindungsgemäßen Vorrichtung zum Entfernen von Zecken die hier beanspruchte Lehre lediglich erläutern, jedoch nicht auf diese Ausführungsbeispiele einschränken.

## Patentansprüche

1. Vorrichtung zum Entfernen von Zecken mit zwei jeweils eine Greifbacke (1) aufweisenden, zumindest teilweise elastischen Greifarmen (2), wobei die Greifarme (2) einander zugeordnet und die Greifbacken (1) aufgrund der Elastizität der Greifarme (2) aufeinander zu und voneinander weg bewegbar sind, wobei sich die Greifarme (2) zwischen einem den Greifbacken (1) abgewandten Verbindungsbereich (3) und den Greifbacken (1) derart kreuzen, daß die Greifbacken (1) mit ihren Eingriffsflächen (4) unter Vorspannung aneinanderliegen und das so gebildete Maul (5) durch Überwindung der Vorspannung öffenbar ist und wobei zwischen dem Verbindungsbereich (3) und dem Kreuzungsbereich (6) der Greifarme (2) ein Betätigungsbereich (7) mit jeweils einem Betätigungselement (8) vorgesehen ist, **dadurch gekennzeichnet,** daß die Greifarme (2) zumindest in dem Verbindungsbereich (3) von einer ein- oder mehrteiligen zylinderförmigen Hülse (12) umgeben sind und daß die Hülse (12) den Betätigungsbereich (7) umgibt und in ihrer Wandung Öffnungen (13) zum Aufnehmen und/oder Betätigen der Betätigungselemente (8) aufweist.

2. Vorrichtung nach Anspruch 1, dadurch gekennzeichnet, daß das Betätigungselement (8) von den Greifarmen (2) abragend etwa halbringförmig ausgebildet ist.

3. Vorrichtung nach Anspruch 2, dadurch gekennzeichnet, daß die Betätigungselemente (8) in entgegengesetzter Richtung im wesentlichen innerhalb der Bewegungsebene der Greifbacken (1) von den Greifarmen (2) abragen und als integrale Bestandteile der Greifarme (2) ausgebildet sind.

4. Vorrichtung nach Anspruch 2 oder 3, dadurch gekennzeichnet, daß sich die Greifarme (2) im Bereich zwischen dem Verbindungsbereich (3) und dem Betätigungsbereich (7) gegeneinander abstützen.

5. Vorrichtung nach Anspruch 4, dadurch gekennzeichnet, daß den beiden Greifarmen (2) jeweils ein Stützelement (19) zum gegenseitigen Abstützen zugeordnet ist, wobei das Stützelement (19) in Form eines Stegs ausgebildet ist, wobei der Steg des einen Greifarms (2) am Steg des anderen Greifarms (2) zur Anlage kommt, und wobei die Stege zumindest geringfügig schwenkbar ineinander eingreifen.

6. Vorrichtung nach einem der Ansprüche 1 bis 5, dadurch gekennzeichnet, daß die Greifbacken (1) im wesentlichen von dem Kreuzungsbereich (6) aus in Richtung Eingriffsflächen (4) divergieren und etwa im Bereich der Eingriffsflächen (4) konvergieren und zwischen dem divergierenden und dem konvergierenden Bereich einen im unbetätigten Zustand der Vorrichtung etwa parallel zu der jeweils anderen Greifbacke (1) angeordneten Abschnitt (9) aufweisen.

7. Vorrichtung nach einem der Ansprüche 1 bis 6, dadurch gekennzeichnet, daß die Hülse (12) die Greifbacken (1) zumindest teilweise umgibt.

8. Vorrichtung nach einem der Ansprüche 1 bis 7, dadurch gekennzeichnet, daß die Hülse (12) eine die Greifbacken (1) umgebende Schutzkappe und an dem den Greifbacken (1) abgewandten Ende (11) eine Abschlußkappe (14) aufweist.

9. Vorrichtung nach einem der Ansprüche 1 bis 8, dadurch gekennzeichnet, daß die einzelnen Bestandteile (14, 16, 17) der Hülse (12) miteinander verschraubbar, verrastbar oder verklemmbar sind.

10. Vorrichtung nach einem der Ansprüche 1 bis 9, dadurch gekennzeichnet, daß die Hülse (12) aus Metall oder Kunststoff besteht.

11. Vorrichtung nach einem der Ansprüche 1 bis 10, dadurch gekennzeichnet, daß die Greifarme (2) in dem Verbindungsbereich (3) an der Hülse (12) festgelegt sind.

12. Vorrichtung nach Anspruch 11, dadurch gekennzeichnet, daß die Greifarme (2) innerhalb zweier in der Abschlußkappe (14) ausgebildeter Öffnungen (15) festgelegt sind.

## Claims

1. Device for removing ticks, having two at least partially resilient gripping arms (2), each of which has a gripping jaw (1), the gripping arms (2) being associated with one another and the gripping jaws (1) being movable towards and away from one another owing to the resilience of the gripping arms (2), the gripping arms (2) intersecting between a connecting region (3), remote from the gripping jaws (1), and the gripping jaws (1) in such a manner that the gripping jaws (1) abut one another with their contacting faces (4) under prestress and the mouth (5) so formed can be opened by overcoming the prestress, and an operating region (7) having a respective operating element (8) being provided between the connecting region (3) and the intersection region (6) of the gripping arms (2), characterised in that the gripping arms (2) are surrounded, at least in the connecting region (3), by a cylindrical sleeve (12) which is in one or more parts and in that the sleeve (12) surrounds the operating region (7) and has, in its wall, openings (13) for receiving and/or operating the operating elements (8).

2. Device according to claim 1, characterised in that the operating element (8), projecting from the gripping arms (2), is in an approximately half-ring form.

3. Device according to claim 2, characterised in that the operating elements (8) project from the gripping arms (2) in opposite directions substantially within the plane of movement of the gripping jaws (1) and are constructed as integral components of the gripping arms (2).

4. Device according to claim 2 or 3, characterised in that the gripping arms (2) are supported against one another in the region between the connecting region (3) and the operating region (7).

5. Device according to claim 4, characterised in that a respective support element (19) is associated with each of the two gripping arms (2) for mutual support, the support element (19) being in the form of a crosspiece, the crosspiece of the one gripping arm (2) abutting the crosspiece of the other gripping arm (2), and the crosspieces engaging in one another at least slightly pivotably.

6. Device according to any one of claims 1 to 5, characterised in that the gripping jaws (1) diverge, starting substantially from the intersection region (6), in the direction towards contacting faces (4) and converge approximately in the region of the contacting faces (4) and have, between the diverging and the converging region, a portion (9) which, when the device is in the non-operated state, is arranged approximately parallel to the respective other gripping jaw (1).

7. Device according to any one of claims 1 to 6, characterised in that the sleeve (12) surrounds the gripping jaws (1) at least partially.

8. Device according to any one of claims 1 to 7, characterised in that the sleeve (12) has a protective cap surrounding the gripping jaws (1), and an end cap (14) on the end (11) remote from the gripping jaws (1).

9. Device according to any one of claims 1 to 8, characterised in that the individual components (14, 16, 17) of the sleeve (12) can be screwed, locked or clamped to one another.

10. Device according to any one of claims 1 to 9, characterised in that the sleeve (12) consists of metal or plastics material.

11. Device according to any one of claims 1 to 10, characterised in that the gripping arms (2) are secured to the sleeve (12) in the connecting region (3).

12. Device according to claim 11, characterised in that the gripping arms (2) are secured inside two openings (15) formed in the end cap (14).

## Revendications

1. Dispositif pour ôter les tiques avec deux bras de préhension (2) au moins partiellement élastiques présentant chacun une mâchoire de préhension (1), où les bras de préhension (2) sont associés l'un à l'autre et où en raison de l'élasticité des bras de préhension (2), les mâchoires de préhension (1) peuvent être rapprochées et être éloignées l'une de l'autre, où les bras de préhension (2) entre une zone de liaison (3) non orientée vers les mâchoires de préhension (1) et les mâchoires de préhension (1) se croisent de manière telle que par leurs faces d'engagement (4), les mâchoires de préhension sont appliquées sous contrainte l'une sur l'autre et que l'orifice (5) ainsi formé peut être ouvert sous l'action d'une force qui est supérieure à la précontrainte, et où entre la zone de liaison (3) et la zone de croisement (6) des bras de préhension (2) est prévue une zone d'actionnement (7) avec chaque fois un élément d'actionnement, caractérisé en ce que du moins dans la zone de liaison (3), les bras de préhension (2) sont entourés d'une douille de forme cylindrique (12) en une ou plusieurs parties et en ce que la douille (12) entoure la zone d'actionnement (7) et présente dans sa paroi des ouvertures (13) pour la réception et/ou l'actionnement des éléments d'actionnement (8).

2. Dispositif suivant la revendication 1, caractérisé en ce que l'élément d'actionnement (8) issu des bras de préhension (2) est de forme sensiblement semi-circulaire.

3. Dispositif suivant la revendication 2, caractérisé en ce que les éléments d'actionnement (8) font, en sens opposé, saillie sur les bras de préhension (2) en substance au sein du plan de mouvement des mâchoires de préhension (1) et font partie intégrante des bras de préhension (2).

4. Dispositif suivant la revendication 2 ou la revendication 3, caractérisé en ce que dans la région entre la zone de liaison (3) et la zone d'actionnement (7), les bras de préhension (2) s'étayent mutuellement.

5. Dispositif suivant la revendication 4, caractérisé en ce qu'à chacun des deux bras de préhension (2) est associé un élément d'appui (19) pour l'étayage mutuel, où l'élément d'appui (19) a la forme d'une nervure et où la nervure d'un des bras de préhension (2) vient d'appliquer sur la nervure de l'autre bras de préhension (2), et où les nervures s'engagent l'une dans l'autre de manière à pouvoir pivoter au moins à un degré réduit.

6. Dispositif suivant l'une quelconque des revendications de 1 à 5, caractérisé en ce que les mâchoires de préhension (1) divergent en substance au niveau de la zone de croisement (6) en direction des faces d'engagement (4) et convergent sensiblement au niveau des faces d'engagement (4) et présentent, entre la zone de divergence et la zone de convergence, un tronçon (9) disposé, lorsque le dispositif n'est pas actionné, sensiblement parallèlement à l'autre mâchoire de préhension (1).

7. Dispositif suivant l'une quelconque des revendications de 1 à 6, caractérisé en ce que la douille (12) entoure au moins partiellement les mâchoires de préhension (1).

8. Dispositif suivant l'une quelconque des revendications de 1 à 7, caractérisé en ce que la douille (12) présente un capuchon de protection entourant les mâchoires de préhension (1) et présente un capuchon de clôture (14) à l'extrémité opposée aux mâchoires de préhension (1).

9. Dispositif suivant l'une quelconque des revendications de 1 à 8, caractérisé en ce que les différents composants (14, 15, 17) de la douille (12) peuvent être réunis par vissage, par encliquetage ou par serrage.

10. Dispositif suivant l'une quelconque des revendications de 1 à 9, caractérisé en ce que la douille (12) est fabriquée en métal ou en matière plastique.

11. Dispositif suivant l'une quelconque des revendications de 1 à 10, caractérisé en ce que dans la zone de liaison, les bras de préhension (2) sont fixés à la douille (12).

12. Dispositif suivant l'une quelconque des revendications de 1 à 11, caractérisé en ce que les bras de préhension (2) sont fixés dans deux ouvertures (15) pratiquées dans le capuchon de clôture (14).
